# EUROPEAN PATENT SPECIFICATION

(11) **EP 2 793 679 B1**
(45) Date of publication and mention of the grant of the patent: **17.05.2017**
(21) Application number: 12809805.0
(22) Date of filing: 19.12.2012
(51) Int. Cl.: A61B 1/307, A61B 1/06, A61B 1/04

(54) **AN ILLUMINATION SYSTEM FOR ENDOSCOPIC APPLICATIONS**
BELEUCHTUNGSSYSTEM FÜR ENDOSKOPISCHE ANWENDUNGEN
SYSTÈME D'ÉCLAIRAGE POUR APPLICATIONS ENDOSCOPIQUES

(30) Priority: 19.12.2011 EP 11194323
(43) Date of publication of application: 29.10.2014
(73) Proprietor: Technical University of Denmark, 2800 Kgs. Lyngby (DK); Frederiksberg Hospital, 2000 Frederiksberg C (DK)
(72) Inventor: LINDVOLD, Lars, DK-2980 Kokkedal (DK); HERMANN, Gregers, DK-2800 Lyngby (DK)
(74) Representative: Zacco Denmark A/S
(86) International application number: PCT/EP2012/076193
(87) International publication number: WO 2013/092740

(56) References cited:
- EP-A1- 2 283 766
- US-A1- 2004 006 276
- US-A1- 2007 285 771
- US-A1- 2011 017 923
- US-B1- 6 665 556

## Description

The present disclosure relates to an endoscope for examining a body cavity and a system for illuminating the region of examination in medical endoscopic application. The present disclosure also relates to a method for illuminating at least a part of the inner surface of a body cavity. A system for photodynamic diagnosis and/or therapy of bladder cancer is furthermore disclosed herein.

### Background of invention

Endoscopy is gaining increased popularity in the medical world because of reduced costs in relation to examination, diagnosis, therapy and surgery and reduced risks, discomfort and nuisance to the patients.

An endoscope typically consists of a rigid or flexible tube with a distal end entering the patient and a proximal end towards the user of the endoscope. A light delivery system is typically provided to illuminate a body cavity or the organ or object under inspection (the region of examination), an optical or electrical relay system for transmitting the image to the viewer, an eyepiece and/or video display for the viewer to see the region of examination, and optionally one or more additional channels to allow entry of medical instruments or manipulators. The light source(s) is normally outside the body and the light is typically directed through the tube via an optical fiber system. The optical relay system can be a lens system in the case of rigid endoscopes and a bundle of optical fibers in the case of a flexible endoscope (fiberscope). Naturally the cross sectional area of an endoscope should be minimal. In an endoscope a CCD based camera may convert the optical image to a digital image. The camera can be located at the proximal end of the endoscope. However, in a digital endoscope a miniature CCD based camera may be located at the distal end of the endoscope whereby the digital image formed in the camera can be transmitted via an electrical connection to the proximal end, thereby eliminating the need for an optical relay system for transmitting images from the distal to the proximal end.

US 2007/285771, US 2011/017923 and EP 2283766 describe traditional endoscopes with one or more light sources for generating fluorescence in the tissue supplemented with a bright light illumination light source for lighting up the tissue with visible light for the surgeon.

US 6,665,556 discloses an apparatus (e.g. an endoscope) for examining tissue using the spectral wing emission therefrom. The document discloses that the tissue is irradiated with a monochromatic light source with a wavelength of at least 600 nm and the spectral wing emission emitted from the tissue is used to characterize the condition of the tissue. According to the document, the spectral wing emissions from different tissue types (e.g., cancer, precancer, normal, fat) induced by visible (specifically, at least 600 nm) to infrared photoexcitation are distinguishable from one another and can be used to characterize a tissue whose condition is unknown.

A common problem encountered in endoscopic examination of e.g. bladders is that urine has a relatively strong absorption in the UV-blue region. Many commercial monochromatic light sources, used for photodynamic diagnosis or other methods for visualizing malignant tissue, emit light in the UV-blue region and thus cause strong green fluorescence in urine that confounds the sensitized fluorescence of the malignant tissue. As urine enters the bladder constantly during examination this problem cannot be avoided and prevents the use of photodynamic diagnosis for bladder cancer to be used in the outpatient department (OPD). Normally an endoscope utilizes two wavelength bands, one bright white light source used for illuminating the bladder with white light and a narrow band of light obtained by optically filtering the white light source for exciting the fluorophore of the photosensitizer. The physician locates the pre-cancerous tissue with the fluorescent light and switches to white light in order to remove the pre-cancerous tissue surgically. Thus, the physician has to switch between the two light sources during examination.

Another problem encountered in diagnostic methods based on fluorescent labeling and photodynamic diagnosis of pre-cancerous tissue in the bladder is photobleaching of the fluorophores used for labeling. Photobleaching is primarily caused by bright blue light sources used for illuminating the bladder. The consequence is that some precancerous tissues may remain undetected by the physician.

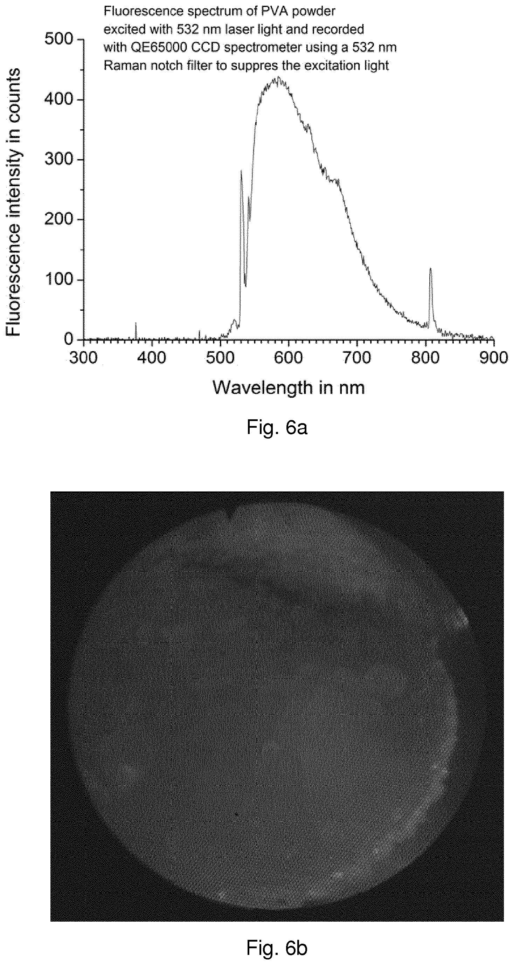

US 2007/285771 discloses an endoscope which comprises up to three light sources marked with reference numbers 8, 9 and 20. Light source 8 is for illuminating a cavity and emits light from 430-460 nm. The light from light source 8 is not meant to induce (auto)fluorescence - the light is instead reflected back from the cavity and collected by the image pick up unit (3/3'). Light source 9 is an excitation light source emitting light at 405 nm. The 405 nm excitation light induces autofluorescence in the natural occurring items in the cavity. The spectrum of the autofluorescence light is shown in the third graph from below in figure 6. Light source 20 is an excitation light source emitting light at 660 nm. The 660 nm excitation light induces fluorescence in a substance administrated to the patient. The spectrum of the fluorescence light is shown in the bottom graph in figure 6.

US 2004/006276 discloses a device which can be used as a cystoscope to generate autofluorescence in the bladder, which in combination with measurements of elastic light scattered images obtained using white light from light source with reference number 80, where the generation of autofluorescence in combination with the elastic scattering imaging data is used for detection of cancerous tissue.

### Summary of invention

To provide for visual inspection inside the body a bright white light source is preferred, but the optical attenuation of the visible light is enormous when white light sources are coupled into a fiber bundle and transmitted to the distal end of the endoscope. Typical examination light sources are therefore high power white light sources, like xenon lamps or metal halide lamps that consume hundreds of watts of power. Bulky liquid core fibers with diameter of up to 8 mm may be used in the endoscope to transport the light, but such liquid core fibers are very costly and extremely vulnerable. One purpose of the invention is therefore to provide a new type of endoscope as defined in claims 1-11. Further disclosed is an illumination system for endoscopic applications comprising at least one substantially monochromatic light source having a predefined central wavelength between 400 and 500 nm or between 500 and 550 nm, an optical transmission path adapted to guide light emanating from the light source to an endoscopic region of examination, and an optical band-rejection filter, wherein the illumination system is adapted to illuminate at least a part of the region of examination by generating autofluorescence in surrounding tissue, and the band-rejection filter is adapted to attenuate at least said light source wavelength to a viewer and wherein said light source is the single light source in the illumination system.

A further example not forming part of the invention relates to an endoscope for examining a body cavity comprising tissue, the endoscope comprising a source of light consisting of a substantially monochromatic light source having a predefined central wavelength between 400 and 550 nm, means for guiding light from the substantially monochromatic light source towards at least a part of the tissue, and at least one band-rejection filter adapted to attenuate at least said central wavelength, wherein the substantially monochromatic light source is configured to generate autofluorescence in the irradiated tissue such that the irradiated tissue is observable, and wherein the endoscope is configured to display at least a part of the irradiated tissue through said band-rejection filter.

A further example not forming part of the invention relates to a method for illuminating at least a part of the inner surface of a body cavity of a subject for a viewer and/or for rendering at least a part of the inner surface of a body cavity of a subject visible to a viewer, the body cavity comprising tissue, the method comprising the steps of providing substantially monochromatic light having a predefined central wavelength between 400 and 550 nm to the body cavity, and generating autofluorescence in at least a part of the tissue in the body cavity by irradiating said tissue with the monochromatic light, the irradiated tissue thereby being illuminated by the autofluorescent light and/or thereby rendering said tissue visible to the viewer. The irradiated tissue may thereby be rendered visible to the viewer, e.g. using an imaging device such as an endoscope, because the irradiated tissue fluoresces due to the generated autofluorescence.

The endoscopes and the illumination systems and the methods of the present disclosure need only have this one light source as the monochromatic light source generates sufficient autofluorescence in the body cavity to allow an observer, e.g. a physician to view the tissue irradiated by the light from the monochromatic light source because the irradiated tissue is being illuminated by the autofluorescence generated in the irradiated tissue, i.e. the tissue fluoresces whereby it becomes visible. Thus, preferably the substantially monochromatic light source is the only light source. Thus, preferably no broadband light source is used for illuminating the endoscopic region of examination, the body cavity, tissue, i.e. preferably, no incandescent light source, no discharge based light sources, no white light source, or no light source obeying Planck's radiation law is used in the endoscopes or illumination systems or methods of the present disclosures.

The present disclosure further relates to an endoscopic system incorporating the illumination system as herein disclosed.

The inventors have observed that the combination of a monochromatic light source (such as a laser light or LED source) and the band rejection filter, preferably a narrow band notch filter, allows the surgeon to use the autofluorescence of the surrounding (healthy) tissue as normal examination light, because the irradiated tissue fluoresces and thereby becomes visible. In an example a 532 nm laser was used, in another example a 525 nm LED was used resulting in an autofluorescence spectrum from surrounding tissue of approx. 550-700 nm, i.e. only the green, yellow and red part of the visible spectrum, but still adequate for discerning the morphology of the tissue. Using the monochromatic light source generated autofluorescence light from the healthy tissue as normal examination light allows the surgeon to skip the use of bulky liquid core light guides and power consuming metal halide lamps or discharge lamps, like xenon lamps, normally used in many endoscopic procedures. Use of a laser or LED as examination light source greatly reduces the footprint of the optical transmission path, because the laser light may be transmitted to the region of examination via a thin optical fiber with a diameter of 0.5 mm. And the power consumption of the excitation light source may also be reduced.

### Description of drawings

The invention will in the following be described in greater detail with reference to the accompanying drawings:
- Fig. 1: shows an image of red PPIX fluorescence of three malignant areas in the bladder excited by blue light and observed through a cystoscope (prior art technique),
- Fig. 2: shows fluorescence spectra of PPIX and urine measured using 395 nm excitation light,
- Fig. 3a-b: shows fluorescence spectra of PPIX and urine measured using 532 nm excitation light,
- Fig. 4: shows the absorption spectrum of PPIX,
- Fig. 5: shows fluorescence spectrum of human fingertip measured using 532 nm excitation light,
- Fig. 6a: shows fluorescence spectra of PVA powder measured using 532 nm excitation light,
- Fig. 6b: shows fluorescence of PVA powder using 532 nm excitation light and observed through a cystoscope,
- Fig. 7a: shows fluorescence spectra of PPIX adsorbed on PVA measured using 532 nm excitation light, and
- Fig. 7b: shows fluorescence of PPIX adsorbed on PVA using 532 nm excitation light and observed through a cystoscope,
- Fig. 8: shows UV-VIS absorption spectrum of urine measured in a 10 mm cuvette,
- Fig. 9: shows the emission spectrum of a 525 nm LED with and without bandpass filter,
- Fig. 10: shows the considerable wear of a Xenon based light source compared to the constant output over time of an LED based light source, and
- Fig. 11: shows an embodiment of an illumination system according to the present disclosure.

### Definitions

**The region of examination** (or the region of interest) is the area or region which is to be endoscopically examined, e.g. a body cavity. In case of bladder examination a cystoscope (typically flexible) is guided through the urinary tract to enter the bladder, which is an example of a body cavity. The region of examination is then the inside of the bladder (or at least a part of it).

**Fluorescence** is the emission of light by a substance that has absorbed light or other electromagnetic radiation of a different wavelength. It is a form of luminescence. In most cases the emitted light has a longer wavelength (Stokes shift), and therefore lower energy, than the absorbed radiation.

**Autofluorescence** is the native fluorescence of biological entities. Cells contain certain molecules, which become fluorescent when excited by UV/Visual radiation of suitable wavelength. This fluorescence emission, arising from endogenous fluorophores, is an intrinsic property of cells and is called autofluorescence which is different from fluorescent signals obtained by adding exogenous markers. Autofluorescence can be problematic in fluorescence microscopy. Light-emitting stains are applied to samples to enable visualization of certain structures. Autofluorescence then may interfere with detection of specific fluorescent signals, especially when the signals of interest are very dim - it causes structures other than those of interest to become visible. In most applications autofluorescence is therefore a problematic issue that must be dealt with and circumvented. However, in a few cases autofluorescence may illuminate the structures of interest, or serve as a useful diagnostic indicator. E.g. cellular autofluorescence can be used as an indicator of cytotoxicity without the need to add fluorescent markers. The autofluorescence of human skin can be used to measure the level of advanced glycation end-products (AGEs), which are present in higher quantities during several human diseases.

**Photosensitizers** are a chemical compounds capable of altering the native photophysical and/or photochemical properties of a substance or cell. Photosensitization is thus the process of transferring the energy of absorbed light. The first step in the photosensitizing process is the absorption of a light photon by the photosensitizer in the ground state and its promotion to the short-lived excited singlet state. The singlet state decays back to the ground state, resulting in the emission of light in the form of fluorescence. Alternatively, the excited singlet state may transfer energy via intersystem crossing (ISC) to the triplet state where the excited state decay into the ground state with emission of a longer wavelength than that of the singlet process also known as phosphorescence. This process typically takes place at time scale 3-6 orders of magnitude longer than that of fluorescence. In the case of photodynamic therapy (PDT) the energy of the excited triplet state is typically transferred to molecular oxygen to create singlet oxygen, a very potent oxidising agent and cell poison. Exogenous photosensitizer is a common term for photosensitizers which are not naturally occurring in the human or animal body and which are added to the subject before examination. Some exogenous photosensitizers can be accumulated in precancerous, malignant and/or fast-growing cells in body and thus used for photodynamic diagnosis (PDD) of e.g. cancer. Exogenous photosensitizers are a key component of photodynamic diagnosis (PDD) used to diagnose cancer and photodynamic therapy (PDT) used to treat cancer.

### Photodynamic diagnosis of bladder cancer

Bladder cancer is identified and resected during endoscopic examination of the bladder through the urethra. A new kind of photodynamic diagnosis (PDD) of bladder cancer (BC) was developed in 2001 where hexaminolevulinate or 5-aminolevulinic acid (5-ALA) is used as precursor to the dye. The aminoacid 5-ALA is metabolized to protoporphyrin IX (PPIX) in the malignant cells which fluoresces at approx. 635 nm when excited with e.g., blue light. During endoscopic examination using a white light source as examination light, the malignant areas become visible as clear red areas when the light is shifted to a blue excitation light source. PDD improves the diagnoses and thus the treatment of bladder cancer substantially. The recurrence rate of bladder cancer within one year after tumor surgery is reduced from 47% to 30% when using PDD.

A problem with this procedure is that yellow urine in the bladder is also excited by the blue light to generate strong green fluorescence. This confounds the fluorescence and the vision in the bladder becomes heavily impaired by the green fluorescence and thus the diagnosis of the malignant tissue. This problem may be reduced by washing the bladder with glycine or saline prior to examination, but the subsequent examination is still a race against time because new urine may be accumulated in the bladder during examination which may make it difficult to identify all bladder tumors. A further object of the invention is therefore to improve the diagnosis of bladder cancer and/or tumors. This object is achieved by a cystoscopic system for photodynamic diagnosis of bladder cancer comprising an endoscopic tube accommodating at least one optical transmission path adapted to guide excitation light and examination light to a region of examination, and an optical relay system adapted to image the region of examination and accommodating an optical band-rejection filter adapted to attenuate at least said excitation light, wherein the excitation light is adapted to excite fluorescence of a photosensitive compound and the wavelength of the excitation light source is greater than 460 nm, more preferably greater than 500 nm and less than 550 nm.

The photosensitive compound is preferably accumulated in precancerous, malignant and/or fast-growing cells in the bladder. The photosensitive compound is preferably selected from the group of porphyrins, such as haematoporphyrin or protoporphyrin, preferably protoporphyrin IX (PPIX). The photosensitive compound is preferably delivered to malignant cells by means of a precursor based on levulinic acid, such as hexaminolevulinate (e.g. Hexvix®), 5-aminolevulinic acid (ALA or 5-ALA) or methyl aminolaevulinate (MAL, e.g. Metvix). Levulinic acid are metabolized to photosensitive PPIX in cells through the intrinsic cellular haem biosynthetic pathway.

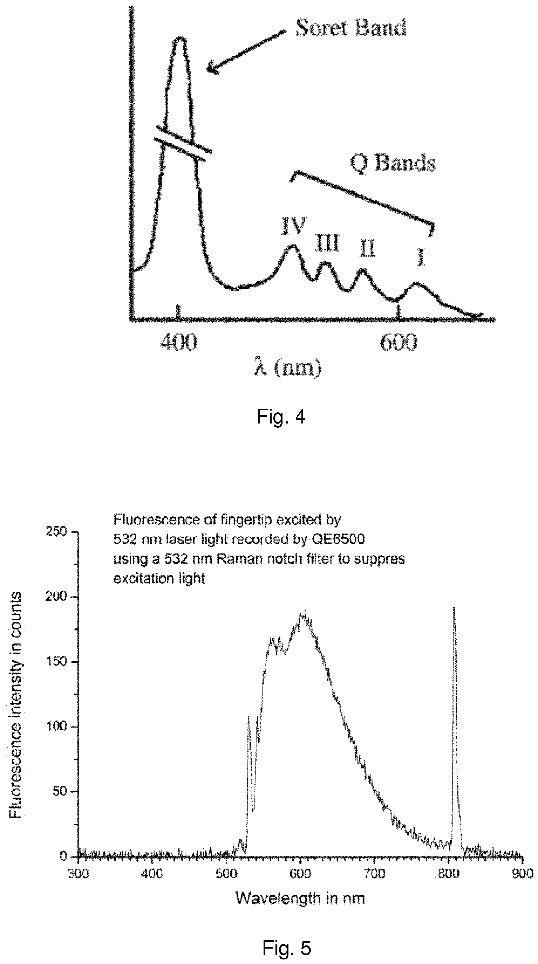

Normally the strongest absorption peak of PPIX around 405 nm is used (the Soret band, see fig. 4). But the inventors have realized that one of the other absorption peaks (the Q bands, see fig. 4) may be used to excite fluorescence of PPIX if a band-rejection filter is used to remove the excitation light from the viewer's line of sight, e.g. a notch filter may be incorporated in the optical relay system or in front of an eyepiece or (the CCD of) a monitoring camera. By using non-blue excitation light fluorescence from urine is avoided. This allows a surgeon using an endoscope in treatment of bladder cancer to improve the visual contrast in photodynamic diagnosis of bladder cancer and carcinoma *in situ* by optical means. This improvement is of particular interest when the patient accumulates urine in the bladder during the examination. The present invention will furthermore make it possible to perform photodynamic diagnosis of bladder cancer in the outpatient department which will greatly reduce the cost of bladder cancer / tumor diagnosis and treatment. The present invention will also improve the treatment of bladder cancer and urotelial tumors of the renal pelvis in the operating theater and reduce the risk of recurrence. After the bladder cancer / tumor has been identified with PDD it must be removed. Today the specific removal is typically provided using a white light examination source to provide visibility for the surgeon. However, under white light the malignant areas are not fluorescing and during removal there is a great risk that not all tumor cells are actually removed. As stated above photobleaching of the fluorophores used for labeling is a problem in PDD. As photobleaching is primarily caused by white light sources the present bright white light sources used for illuminating body cavities like the bladder is a serious issue. Thus, the present diagnosis of bladder cancer is not only a race against the recurrence of urine, but also a race against elimination of the fluorophores due to photobleaching. The consequence is that some tumor cells may be overlooked. The present invention utilizing a single light source as both excitation and illumination light source will make the malignant areas fluorescing during removal and thereby make it possible for the surgeon to better follow the removal of the tumor cells to make sure they are completely removed. And what is equally important is that the monochromatic light source, i.e. a green light source, photobleaches the fluorophores at a significantly lower rate than the conventional blue light source. Looking ahead the present invention will make it possible to move the treatment of bladder cancer (i.e. removal of tumors) to the outpatient department, i.e. the diagnosis and immediate treatment can be combined to a single quick procedure in the outpatient department with improved quality of life for the patients and greatly reduced cost for the health care system and the society as the result.

If the patient under examination has a bladder infection the green fluorescence from urine when using blue light excitation is even stronger due to fluorescence from bacteria originating from the bladder infection. This makes it even more difficult to use the blue light as excitation light source. As stated above the present illumination system may be incorporated in an endoscopic system, in particular a cystoscopic system for examination of the urinary tract and/or the bladder, when a patient has a bladder infection. A further example not forming part of the invention therefore relates to a cystoscopic system comprising an endoscopic tube and incorporating the illumination system as herein described, wherein the optical transmission path is incorporated in the endoscopic tube, said endoscopic tube further comprising an optical relay system adapted to image the region of examination, wherein the predefined central wavelength of the illumination system is greater than 460 nm, such as greater than 470 nm, such as greater than 480 nm, such as greater than 490 nm, such as greater than 500 nm, such as greater than 510 nm. The predefined central wavelength of the illumination system is preferably less than 550 nm.

Please note that as stated above the use of a non-blue excitation light source may be incorporated in existing cystoscopic systems. However, it will be even better if the photodynamic diagnosis of bladder cancer is combined with the illumination system according to the present disclosure. Thus, a further example not forming part of the invention relates to a cystoscopic system for photodynamic diagnosis of bladder cancer comprising an endoscopic tube and incorporating the above mentioned illumination system, wherein the optical transmission path is incorporated in the endoscopic tube, said endoscopic tube further comprising an optical relay system adapted to image the region of examination, wherein the illumination system is adapted to excite fluorescence a photosensitive compound and wherein the predefined central wavelength of the illumination system is greater than 460 nm, such as greater than 470 nm, such as greater than 480 nm, such as greater than 490 nm, such as greater than 500 nm, such as greater than 510 nm. The predefined central wavelength of the illumination system is preferably less than 550 nm. A further embodiment relates to an endoscope as herein disclosed for photodynamic diagnosis of bladder cancer and/or renal pelvis tumor or cancer, wherein the source of light is adapted to excite fluorescence of an exogenous photosensitizer. Thereby a single light source functions as both excitation light source for the exogenous photosensitizer used in PDD and examination light source by the autofluorescence generated in surrounding tissue.

### Detailed description of invention

As stated above the present disclosure relates to an endoscope for examining a body cavity comprising tissue, the endoscope comprising a source of light consisting of a substantially monochromatic light source having a predefined central wavelength between 400 and 550 nm, means for guiding light from the substantially monochromatic light source towards at least a part of the tissue, and at least one band-rejection filter adapted to attenuate at least said central wavelength, wherein the substantially monochromatic light source is configured to generate autofluorescence in the irradiated tissue such that the irradiated tissue is observable, and wherein the endoscope is configured to display at least a part of the irradiated tissue through said band-rejection filter.
The present disclosure also relates to a method for illuminating at least a part of the inner surface of a body cavity of a subject for a viewer, the body cavity comprising tissue, the method comprising the steps of providing substantially monochromatic light having a predefined central wavelength between 400 and 550 nm to the body cavity, and generating autofluorescence in at least a part of the tissue in the body cavity by irradiating said tissue with the monochromatic light, the irradiated tissue thereby being illuminated by the autofluorescent light.

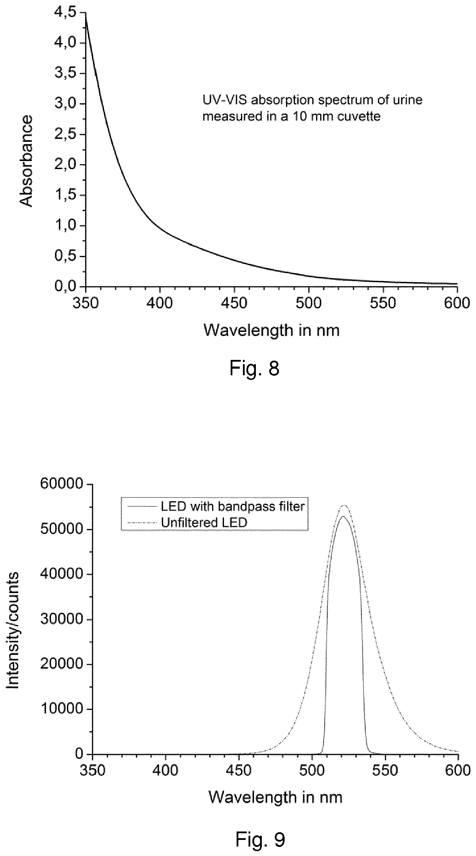

In various examples the endoscope is rigid or flexible. The endoscope may be a videoscope, fiberscope, video endoscope, cystoscope, bronchoscope, laparoscope etc. The choice of central wavelength of the substantially monochromatic light source is a key aspect of the present invention. The lower limit is selected such that absorption and possibly fluorescence from bodily fluids is avoided. In the bladder the possible presence of urine causes that the lower limit of the central wavelength is, according to the invention, 500 nm to avoid absorption in and fluorescence from urine. This can be seen from the absorption spectrum for urine as shown in fig. 8. However, in other body cavities or other regions of examination the lower limit of the central wavelength may be around 400 nm. The upper limit of the central wavelength is selected such that the generated autofluorescence has a suitable spectrum for making the irradiated tissue observable. A higher central wavelength reduces the visible portion of the autofluorescence spectrum as seen from the spectra shown in fig. 5 and 6a which make the irradiated tissue more difficult to observe for a viewer. Thus, according to the invention, an upper limit of 550 nm is selected, thereby still providing a suitable autofluorescence spectrum from the irradiated tissue such that the irradiated tissue is rendered visible and observable for a viewer.

The illumination system according to the present disclosure may be incorporated in endoscopes with eyepieces with the band-rejection filter placed in front of the eyepiece or somewhere in the optical relay system, in endoscopes where the monitoring is provided via monitors/display and provided by means of a camera, with the band-rejection filter placed in front of the camera or the CCD of the camera. The illumination system according to the present disclosure may be incorporated in (digital) endoscopes where the imaging device (e.g. a CCD) is located at the distal end of the endoscope. The band-rejection filter is then placed in front of the imaging device. According to the invention the excitation light is generated by a single monochromatic light source having a predefined central wavelength. In one embodiment of the invention the light source is a laser, such as a fibre coupled laser, a fibre laser, a solid state laser, a diode pumped solid state laser, a light emitting diode (LED) or a semiconductor laser. The light source may be adapted to emit continuous wave (CW) light.
A laser light source has the advantage that it can be directed from an external location to the distal end of endoscope through a very thin optical fiber thereby minimizing the cross-sectional area of the endoscope. Means for spreading out the light out of the endoscope, such as a diffuser, may then be necessary to provide a suitable cone of light out the endoscope to irradiate the tissue that is displayed in the endoscope.

An LED has the advantage that it can be retro-fitted to present endoscopes because light from an LED can be guided through the existing optical transmission path that is currently guiding the light from presently used white light sources. A micro LED adapted to be mounted in the distal end of an endoscope may also be provided eliminating the need for an optical transmission path for the illumination light through the endoscope.
The substantially monochromatic light source may have an emission spectrum with a FWHM of between 1 and 50 nm, thereby approx. ranging from a laser source to an LED. Thus, in a further example the emission spectrum of the substantially monochromatic light source has a FWHM of less than 50 nm, or less than 45 nm, or less than 40 nm, or less than 35 nm, or less than 30 nm, or less than 25 nm, or less than 20 nm, or less than 15 nm, or less than 10 nm, or less than 8 nm, or less than 6 nm, or less than 4 nm, or less than 3 nm, or less than 2 nm, or less than 1 nm. When using an LED it may be an advantage to combine with a bandpass filter to narrow the emission spectrum of the light source. This is exemplified in fig. 9 showing the emission spectrum of an unfiltered LED centred at 525 nm and the same LED with a bandpass filter. The FWHM of the unfiltered LED is approx. 40 nm, however the spectrum of the LED actually covers a span from 450 nm to 600 nm. This may not be desirable because the "blue" 450-500 nm range is possibly unwanted due to stimulation of green fluorescence from e.g. urine and the "red" 550-600 nm range will confound the native fluorescence of the tissue. Consequently a bandpass filter can be inserted, e.g. in the optical path between the light source and the endoscope. The bandpass filter reduces the FWHM to approx. 25 nm thereby reducing the lower wavelength light that may induce fluorescence in e.g. urine and also reducing the longer wavelength light that may be allowed through the band-rejection filter, i.e. the filter maximises the desired sensitised fluorescence from cancerous tissue as well as the native autofluorescence of the healthy tissue. A further advantage of a bandpass filter is that it may be designed so that the bandpass filter for the light source and the band-rejection filter for the viewer are adapted to match each other closely, however preferably with no overlap between the two filters.

The means for guiding light from the substantially monochromatic light source towards at least a part of the tissue may comprise an optical transmission path through or along the endoscope if the light source is located externally or at the distal end of the endoscope. These means for guiding light may comprise optics adapted to direct the light towards tissue such that the irradiated tissue corresponds to the tissue displayed in the endoscope.

The substantially monochromatic light source is configured to generate autofluorescence in the irradiated tissue such that the irradiated tissue is rendered observable, i.e. the irradiated tissue is visible for a viewer because the generated autofluorescence preferably comprises light in the visible spectrum. This is typically provided with a sufficient power from the light source. Measurements on a commercially available cystoscope for PDD has shown that the power in the liquid core fiber that connects to the distal end of the cystoscope is between 9 and 150 mW/cm² for the blue excitation light at 430 nm, and 25 to 300 mW/cm² for the Xenon based white illumination light with a central wavelength at 550 nm. The blue and white light sources can be replaced by a single green LED based light source with a central wavelength of 525 nm with a power of 25 mW/cm² measured in the fiber that connects to the proximal end of the cystoscope. This single green light source provides sufficient power to excite the exogenous photosensitizer in PDD and to generate autofluorescence in the irradiated tissue such the irradiated tissue is observable in the cystoscope. In general sufficient autofluorescence for rendering the irradiated tissue observable is generated within a large power range. If the power from the light source is measured out of the distal end of the endoscope sufficient and suitable autofluorescence will be generated within a power range from approx. 1 mW/cm² to approx. 1000 mW/cm². The required power depends on the configuration of the endoscope. If using a digital camera it depends on the sensitivity of the sensor. A high sensitivity reduces the power requirements. A camera located in the distal end of the endoscope, i.e. a digital endoscope, typically also reduces the power requirements. Thus, the power of the substantially monochromatic light source measured out of the distal end endoscope or out of the optical transmission path of the illumination system may be greater than 1 mW/cm², or greater than 10 mW/cm², or greater than 20 mW/cm², or greater than 30 mW/cm², or greater than 40 mW/cm², or greater than 50 mW/cm², or greater than 75 mW/cm², or greater than 100 mW/cm², or greater than 200 mW/cm², or greater than 300 mW/cm², or greater than 500 mW/cm², or greater than 700 mW/cm².

Further, the power of the substantially monochromatic light source measured out of the distal end endoscope or out of the optical transmission path of the illumination system may be less than 1000 mW/cm², or less than 900 mW/cm², or less than 800 mW/cm², or less than 700 mW/cm², or less than 600 mW/cm², or less than 500 mW/cm², or less than 400 mW/cm², or less than 300 mW/cm², or less than 200 mW/cm², or less than 100 mW/cm², or less than 90 mW/cm², or less than 80 mW/cm², or less than 70 mW/cm², or less than 60 mW/cm², or less than 50 mW/cm², or less than 40 mW/cm², or less than 30 mW/cm², or less than 20 mW/cm², or less than 10 mW/cm², or less than 5 mW/cm², or less than 4 mW/cm², or less than 2 mW/cm².

In a further example not forming part of the invention more than one substantially monochromatic light source, each having a predefined central wavelength, may be used. Each light source may then generate an autofluorescent spectrum from surrounding tissue that may combine to provide examination light of a broader wavelength band than what can be provided from a single light source. The resulting examination light may then be a combination of visible light which can be seen by the naked eye and infrared light which provide greater penetration depth into the tissue and which can be observed by digital imaging means, such as a CCD, and converted to a visible signal in a monitor. There are further advantages of using non-blue excitation light in bladder cancer and urotelial renal pelvis cancer/tumour diagnosis. Upon illumination, all photosensitizers are chemically modified or even degraded. In photodynamic therapy (PDT) reactive oxygen species (ROS) are formed, which oxidize intracellular molecules and thereby destroy cells. In PDD photobleaching occurs where the chemical change is less profound. But photobleaching can be identified by a lowering of the fluorescence emission. Thus, during the PDD examination the photosensitizer is "burned off" with a decreasing fluorescence emission as the result. Thus, small malignant areas may not be identified because the fluorescence disappears concurrently with modification of the photosensitizer. It turns out that the photobleaching when using blue light is significantly higher than if excitation light with a longer wavelength is used. Photobleaching of the photosensitizer may be even more reduced if pulsed light is used. The surgeon will still be able to see the fluorescing malignant areas in pulsed light, and with pulsed light the duty cycle is lower than for CW light, thus the photobleaching will decrease. In a further embodiment of the invention the light source may therefore be adapted to emit pulsed light. And as autofluorescence is an almost instantaneous phenomenon, the excitation light source may still be used as examination light source.

Thus, in one example the duty cycle of the light source is less than 100%, such as less than 90%, such as less than 80%, such as less than 70%, such as less than 60%, such as less than 50%, such as less than 40%, such as less than 30%, such as less than 20%, such as less than 10%, such as less than 5%, such as less than 2%, such as less than 1%.

In one example the repetition rate of the light source is greater than 1 Hz, such as greater than 10 Hz, such as greater than 100 Hz, such as greater than 1 kHz, such as greater than 10 kHz, such as greater than 100 kHz, such as greater than 1 MHz, such as greater than 10 MHz, such as greater than 100 MHz, such as greater than 1 GHz, such as greater than 10 GHz, such as greater than 100 GHz.

In one example the pulse width of the light source is less than 1 second (s), such as less than 0.1 s, such as less than 0.01 s, such as less than 1 ms, such as less than 0.1 ms, such as less than 0.01 ms, such as less than 1 µs, such as less than 0.1 µs, such as less than 0.01 µs, such as less than 1 ns, such as less than 0.1 ns, such as less than 0.01 ns, such as less than 1 fs.

In one example the autofluorescence spectrum generated in tissue comprises visible light. Thus, the predefined central wavelength may be greater than 300 nm, such as greater than 310, such as greater than 320, such as greater than 330, such as greater than 340, such as greater than 350, such as greater than 360, such as greater than 370 nm, such as greater than 380 nm, such as greater than 390 nm, such as greater than 400 nm, such as greater than 410, such as greater than 420, such as greater than 430, such as greater than 440, such as greater than 450, such as greater than 460, such as greater than 470 nm, such as greater than 480 nm, such as greater than 490 nm, such as greater than 500 nm, such as greater than 510 nm, such as greater than 520 nm, such as greater than 530 nm, such as greater than 540 nm, such as greater than 550 nm, such as greater than 560 nm, such as greater than 570 nm, such as greater than 580 nm, such as greater than 590 nm, such as greater than 600 nm.

In a further example the predefined central wavelength is between 500 and 505 nm, or between 505 and 510 nm, or between 510 and 515 nm, or between 515 and 520 nm, or between 520 and 530 nm, or approx. 525 nm, or between 525 and 530 nm, or between 530 and 535 nm, or between 535 and 540 nm, or between 540 and 545 nm, or between 545 and 550 nm.
The predefined central wavelength may be between 300 and 400 nm, such as between 400 and 500 nm, such as between 500 and 600 nm, such as between 350 and 640 nm, such as between 500 and 640 nm, such as between 500 and 600 nm, such as between 500 and 590 nm, such as between 500 and 580 nm, such as between 510 and 580 nm, such as between 500 and 570 nm, such as between 500 and 560 nm, such as between 500 and 550 nm according to the invention, such as between 500 and 540 nm, such as between 510 and 540 nm, such as between 520 and 580 nm, such as between 510 and 560 nm, such as between 520 and 570 nm, such as between 527 and 537 nm, such as between 530 and 534 nm, such as between 531 and 533 nm, such as approx. 532 nm. With a blue laser the resulting autofluorescence in the tissue includes the greater part of the visible spectrum, and a blue laser may therefore constitute a suitable choice of wavelength. However, short wavelength light has less penetration depth into the tissue. If a longer wavelength laser is used the penetration depth into the tissue is higher. Using a green laser may constitute a good compromise in order to generate suitable examination light at the region of interest. Using a longer wavelength than green results in that the autofluorescence spectrum will be shifted toward the infrared and the visible part of the resulting autofluorescence spectrum is thus minimized, i.e. the surgeon will not be able to see the light with the naked eye. However a normal camera is sensitive to near-infrared light. According to an embodiment of the invention, the optical transmission path is preferably incorporated in some sort of wire or cable. In one embodiment of the invention the diameter of the wire or cable incorporating the optical transmission path is less than 2 mm, more preferably less than 1.5 mm, more preferably less than 1 mm, more preferably less than 0.9 mm, more preferably less than 0.8 mm, more preferably less than 0.7 mm, more preferably less than 0.6 mm, more preferably less than 0.5 mm, more preferably less than 0.4 mm, more preferably less than 0.3 mm, more preferably less than 0.25 mm. The optical transmission path may comprises at least one optical waveguide, such as an optical fibre, such as a single mode fibre, or preferably a multimode fibre.

The band-rejection filter may be a narrow band rejection filter, such as, according to an embodiment of the invention, a notch filter, preferably a Raman notch filter, also known as a rugate filter. Another example of a narrow band rejection filter that can be used is a Fabry-Pérot etalon. In the preferred embodiment of the invention the rejection band of the band-rejection filter comprises the light source wavelength; preferably the rejection band of the band-rejection filter is centred on the central light source wavelength. According to an embodiment of the invention, the rejection band of the filter may be less than 20 nm, more preferably less than 15 nm, more preferably less than 12 nm, more preferably less than 10 nm, more preferably less than 8 nm, more preferably less than 6 nm, more preferably less than 4 nm, more preferably less than 2 nm.
The band-rejection filter may also be designed such that it blocks wavelengths below around the central wavelength of the monochromatic light source and allows wavelengths above this wavelength. In the example with the LED light source shown in fig. 9 with a central wavelength at 525 nm and equipped with a bandpass filter, the band-rejection filter could be adapted to block wavelengths below around 540-550 nm and allow wavelengths above, thereby blocking the monochromatic light source for the viewer but allowing the generated autofluorescence light such that irradiated tissue is visible for the viewer.
The band-rejection filter is preferably adapted to attenuate said light source wavelength by more than 10 dB, preferably by more than 20 dB, preferably by more than 30 dB, preferably by more than 40 dB, preferably by more than 50 dB, most preferably by more than 60 dB.
A further embodiment of the disclosure comprises means for removing cancer cells and/or tumours. An exogenous photosensitizer accumulated in precancerous, malignant and/or fast-growing cells will cause fluorescence when irradiated by the presently disclosed light source. These fluorescent areas can be removed by surgical means and/or by optical means, e.g. a laser burning off the fluorescing areas or by means of PDT. In the case of an optical solution means for varying the output power of the light source can be provided.

### Method of illuminating a body cavity and use of an endoscope herein

As previously mentioned a method not forming part of the inventions for illuminating at least a part of the inner surface of a body cavity of a subject for a viewer and/or for rendering at least a part of the inner surface of a body cavity of a subject visible to a viewer, the body cavity comprising tissue, the method comprising the steps of providing substantially monochromatic light having a predefined central wavelength between 400 and 550 nm to the body cavity, and generating autofluorescence in at least a part of the tissue in the body cavity by irradiating said tissue with the monochromatic light, the irradiated tissue thereby being illuminated by the autofluorescent light and/or thereby rendering said tissue visible to the viewer. The irradiated tissue may thereby be rendered visible to the viewer, e.g. using an imaging device such as an endoscope, because the irradiated tissue fluoresces due to the generated autofluorescence. The present inventors have surprisingly found that a substantially monochromatic light source suffices as illumination light source in a body cavity as autofluorescence is generated in the irradiated tissue because the irradiated tissue thereby becomes observable, i.e. visible, for a viewer. I.e. the generated autofluorescent light preferably illuminates a part of the surface of the body cavity for visual inspection of the viewer. This eliminates the need for guiding a broadband white light source to the body cavity. Thus, the substantially monochromatic light source is preferably the only light source that irradiates tissue in the body cavity. Thus, the generated autofluorescent light is preferably the only light source that illuminates the body cavity. Preferably the predefined central wavelength is between 450 and 550 nm, more preferably between 500 and 550 nm.

In a further example the predefined central wavelength is greater than 410 nm, or greater than 420 nm, or greater than 430 nm, or greater than 440 nm, or greater than 450 nm, or greater than 460 nm, or greater than 470 nm, or greater than 480 nm, or greater than 490 nm, or greater than 500 nm, or greater than 510 nm, or greater than 520 nm, or greater than 530 nm, or greater than 540 nm.

In a further example embodiment the predefined central wavelength is less than 540 nm, or less than 530 nm, or less than 520 nm, or less than 510 nm, or less than 500 nm.

In a further example the predefined central wavelength is between 500 and 505 nm, or between 505 and 510 nm, or between 510 and 515 nm, or between 515 and 520 nm, or between 520 and 530 nm, or approx. 525 nm, or between 525 and 530 nm, or between 530 and 535 nm, or between 535 and 540 nm, or between 540 and 545 nm, or between 545 and 550 nm.

In a further example the substantially monochromatic light is optically blocked for the viewer, e.g. by means of a band rejection filter.

One further example comprises the step of administering an exogenous photosensitizer to said subject, said photosensitizer being adapted to accumulate in precancerous, malignant, and/or fast-growing cells and said photosensitizer being able to fluoresce when subjected to said monochromatic light source. Thus, a single monochromatic light source provides fluorescence of the photosensitizer and at the same time makes the irradiated tissue observable for a viewer due to the generated autofluorescence in the irradiated tissue.

A further example not forming part of the invention relates to the use of an endoscope in the method described above.

Yet a further example not forming part of the invention relates to an endoscope as herein disclosed for use in illumination of at least a part of the inner surface of a body cavity of a subject. The use may further comprise the step of examining the body cavity, a body cavity such as the bladder. The use may further comprise the step of diagnosing bladder cancer, e.g. by means of PDD. Thus, the use may use further comprise the step of administrating an exogenous photosensitizer to the patient, said photosensitizer being adapted for being accumulated in precancerous, malignant and/or fast-growing cells in the bladder and said photosensitizer adapted to fluoresce when irradiated by the monochromatic light source.

### Method of diagnosing and/or treatment of bladder cancer

In a further example not forming part of the invention relates to a method for photodynamic diagnosis of bladder cancer or tumour and/or urotelial renal pelvis cancer or tumour comprising
- delivering a photosensitive compound to a region of examination,
- guiding excitation light to the region of examination by means of an endoscope, wherein the excitation light is adapted to excite fluorescence of the photosensitizing dye, and the wavelength of the excitation light source is greater than 460 nm, such as greater than 470 nm, such as greater than 480 nm, such as greater than 490 nm, such as greater than 500 nm, such as greater than 510 nm, and
- imaging the region of examination by means of said endoscope, wherein the wavelength of the excitation light is blocked for a viewer.

In further embodiments the presently disclosed method may incorporate any of the features as herein described.

### Detailed description of Drawings

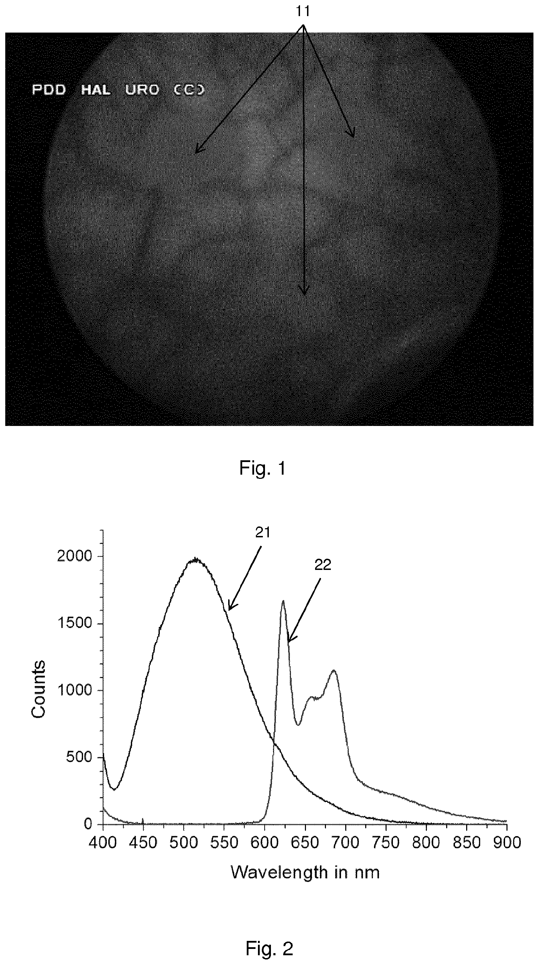

Bladder cancer and tumors is identified and resected during endoscopic examination of the bladder through the urethra. The presently known photodynamic diagnosis (PDD) of bladder cancer utilizes fluorescence of PPIX in malignant cells where e.g. Hexvix® is used as the commercially available precursor to the photosensitizer. PPIX fluoresces at approx 635 nm (red light) when excited with blue light. During endoscopic examination using the malignant areas become visible as clear red areas when the blue excitation light is used. Fig. 1 shows an image of red PPIX fluorescence of three malignant areas 11 in the bladder excited by blue light and observed through a flexible video cystoscope. The malignant areas in fig. 1 might be difficult to see in a black and white reproduction.

In relation to bladder cancer the present invention allows the user of the cystoscope to use a laser with a wavelength longer than blue light, e.g. green light, to excite the dye used in PDD without exciting urine to fluoresce. This has been tested with a 532 nm laser and a very narrow band rejection filter known as a Raman rejection filter or Raman notch filter. It may also be referred to as a rugate filter. This particular filter is characterized by an extremely high rejection ratio (1:1.000.000) of light within a very narrow spectral band (5-10 nm). For practical applications a 532 nm laser and a corresponding 532 nm Raman rejection filter are commercially available, but the present invention may be carried out using other wavelengths of light and filter as defined in the claims.

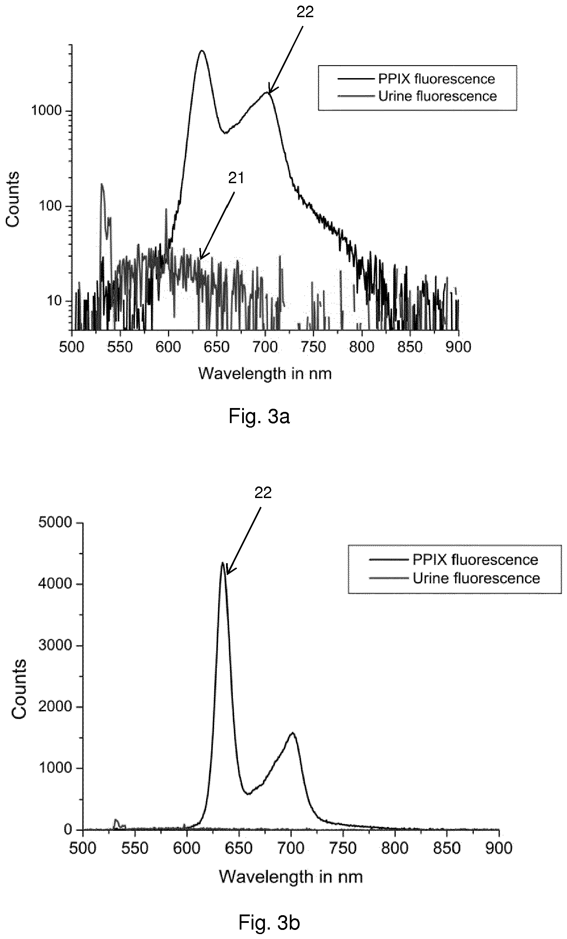

Fig. 2 shows fluorescence spectra of urine 21 and PPIX 22 of in vitro samples (0.6 mg PPIX dissolved in 10 ml Millipore water (18,2 MΩ/cm)) measured using 395 nm excitation light and an Ocean Optics QE65000 spectrometer. Fig. 3 shows fluorescence spectra of urine 21 and PPIX 22 (0.6 mg PPIX dissolved in 10 ml Millipore water (18,2 MΩ/cm)) measured using 532 nm excitation light and an Ocean Optics QE65000 spectrometer with a Raman narrow band (< 10 nm) rejection filter (OD 6). In fig. 3a a log scale is used, in fig. 3b a linear scale is used. The spectra in figs. 2 and 3 clearly show that the fluorescence of urine when excited by 532 nm light was approx.

1000 times weaker than the PPIX fluorescence as shown in the attached spectra. Thus, when using a green light excitation source the fluorescence from urine is avoided while the fluorescence from PPIX is still pronounced.

Fig. 4 shows the absorption spectrum of PPIX with the strong Soret band around 400 nm as the normal absorption band used in PPD of bladder cancer. However, PPIX also has weaker absorption peaks around the Q-bands from approx. 500 to 620 nm.

Fig. 5 shows a fluorescence spectrum of a human fingertip measured using 532 nm excitation light, and an Ocean Optics QE65000 spectrometer with a Raman narrow band (< 10 nm) rejection filter. This fluorescence spectrum of skin was recorded to demonstrate that the spectral distribution of the autofluorescence of skin and the fluorescence of PPIX is different, which can be seen by comparing fig. 3b and fig. 5. The autofluorescence spectrum of tissue as depicted in fig. 5 may be used as excitation light source during endoscopic examination, thereby avoiding the costly and bulky white light sources in normal endoscopes. There is sufficient visible light in the spectrum from approx. 550 to 700 nm for this light to function as direct examination light which is visible the naked eye of the surgeon.

The polymer material PVA has been identified to mimic the autofluorescence of human tissue when excited by 532 nm laser light. This can be seen from fig. Fig. 6a showing fluorescence spectra of PVA powder measured using 532 nm excitation light, and an Ocean Optics QE65000 spectrometer with a Raman narrow band (< 10 nm) rejection filter. Comparing the spectra in fig. 5 (human tissue) and fig. 6a (PVA powder) they show a high degree of similarity. Fig. 6b shows the fluorescence of PVA powder using 532 nm excitation light and observed through a cystoscope.

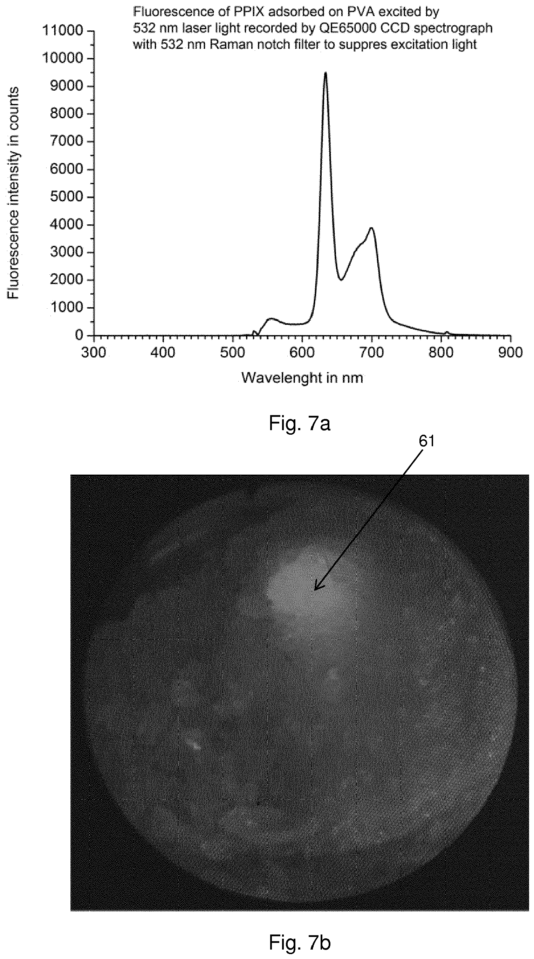

Fig. 7a shows fluorescence spectra of PPIX adsorbed on PVA measured using 532 nm excitation light and an Ocean Optics QE65000 spectrometer with a Raman narrow band (< 10 nm) rejection filter to suppress the excitation light. Fig. 7b shows fluorescence of PPIX adsorbed on PVA using 532 nm excitation light and observed through a cystoscope using fibre optical guiding of laser light to the area of observation. The area 61 where PPIX is adsorbed is clearly visible in the cystoscope as a red spot 61 (bright spot in a black and white reproduction). The conclusion is that when using dyes in PDD of bladder cancer that metabolise to PPIX in malignant cells the malignant areas will be visible when using a light source with a wavelength longer than the normal blue light and fluorescence from urine will be avoided. The problem with the yellow urine on the blue light for PDD can thereby be completely removed by the present invention.

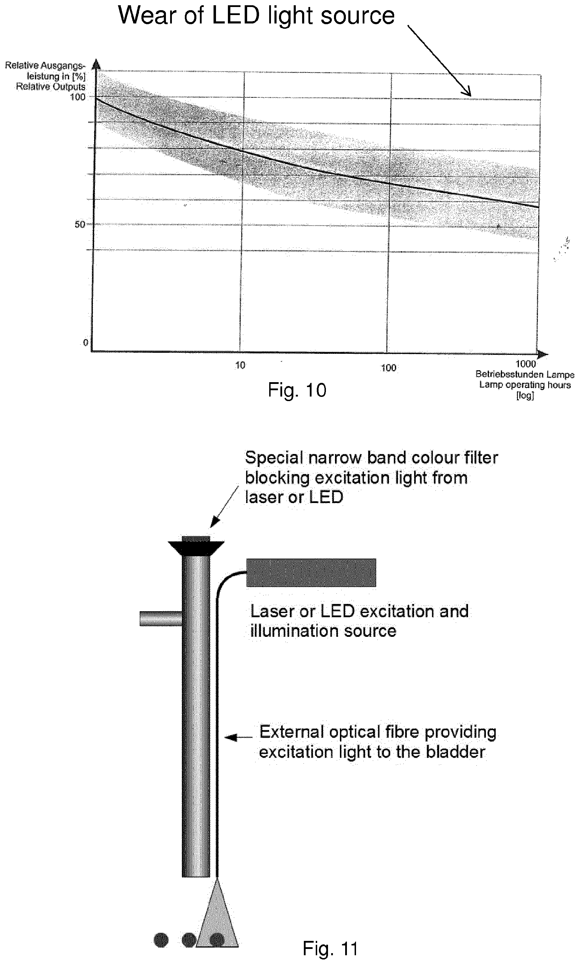

Fig. 11 shows an example of the presently disclosed illumination system for endoscopic application. It shows an example of how an illumination system can be incorporated in an existing commercially available endoscope adapted for examination of the bladder. An external laser or LED green light source is guided to the distal end of the endoscope via an optical fiber that is guided inside a channel in the endoscope, a channel that is normally used for surgical instruments. The green light is emitted from the distal end of the endoscope towards tissue in the bladder. A band-rejection filter is located in the proximal end of the endoscope for blocking the green excitation light from the light source. An exogenous photosensitizer administrated to the patient in combination with the green light cause malignant areas to fluoresce as red spots that can be displayed in the endoscope. The green light also generates autofluorescence in the irradiated tissue thereby rendering the irradiated tissue visible to be displayed in the endoscope. Thus, malignant areas are visible concurrently with the surrounding tissue due to the single green light source. A single green light source thereby replaces the two normal light sources (i.e. white light for illumination + blue light for excitation) with the further advantage that malignant areas and surrounding tissue are visible concurrently.

## Claims

1. An endoscope for examining a body cavity comprising tissue and bodily fluids, the endoscope comprising an illumination system for endoscopic applications in the body cavity, the illumination system comprising:
- a monochromatic light source having a predefined central wavelength between 500 and 550 nm,
- an optical transmission path adapted to guide light emanating from the light source to an endoscopic region of examination, and
- at least one optical band-rejection filter,
wherein the endoscope further comprises means for guiding light from the monochromatic light source towards at least a part of the tissue, said means comprising the optical transmission path, the endoscope being configured to display at least a part of the irradiated tissue through said band-rejection filter,
wherein said at least one band-rejection filter is adapted to attenuate at least said light source wavelength for a viewer, the band-rejection filter being adapted to attenuate said light source wavelength by more than 10 dB, preferably by more than 20 dB, preferably by more than 30 dB, preferably by more than 40 dB, preferably by more than 50 dB, most preferably by more than 60 dB, and
**characterized in that** said light source is the single light source in the endoscope.

2. The endoscope according to claim 1, wherein said light source is a laser, such as a fibre coupled laser, a fibre laser, a solid state laser, a diode pumped solid state laser, a light emitting diode (LED) or a semiconductor laser, and said light source adapted to emit continuous wave (CW) light or pulsed light.

3. The endoscope according to any preceding claim, wherein the optical transmission path is incorporated in a cable or wire with a diameter less than 3 mm, more preferably less than 2 mm, more preferably less than 1.5 mm, more preferably less than 1 mm, more preferably less than 0.9 mm, more preferably less than 0.8 mm, more preferably less than 0.7 mm, more preferably less than 0.6 mm, more preferably less than 0.5 mm, more preferably less than 0.4 mm, more preferably less than 0.3 mm, more preferably less than 0.25 mm.

4. The endoscope according to any preceding claim, wherein the rejection band of the band-rejection filter comprises the light source wavelength, preferably the rejection band of the band-rejection filter is centred at the light source wavelength.

5. The endoscope according to any preceding claim, wherein the rejection band of the filter is less than 20 nm, more preferably less than 15 nm, more preferably less than 12 nm, more preferably less than 10 nm, more preferably less than 8 nm, more preferably less than 6 nm, more preferably less than 4 nm, more preferably less than 2 nm.

6. The endoscope according to any preceding claim, wherein the band-rejection filter is a notch filter, preferably a Raman notch filter.

7. The endoscope according to any preceding claim, wherein the monochromatic light source is located at the proximal end of the endoscope or externally.

8. The endoscope according to any preceding claim, further comprising at least one camera and/or comprising an array of sensor elements.

9. The endoscope according to claim 8, wherein camera and/or the array of sensor elements are located either
- at the distal end of the endoscope, or
- at the proximal end of the endoscope.

10. The endoscope according to any preceding claim, wherein said light source is a LED and wherein the endoscope further comprises a bandpass filter for narrowing the spectral output of the LED.

11. The endoscope according to any preceding claim for photodynamic diagnosis of bladder cancer and/or renal pelvis tumor or cancer, wherein the source of light is adapted to excite fluorescence of an exogenous photosensitizer, wherein the exogenous photosensitizer is accumulated in precancerous, malignant and/or fast-growing cells in the bladder and/or renal pelvis, and wherein the exogenous photosensitizer is based on porphyrins, such as haematoporphyrin or protoporphyrin, such as protoporphyrin IX (PPIX) and wherein the exogenous photosensitizer is administrated by means of a photosensitizing dye based on hexaminolevulinate, 5-aminolevulinic acid (ALA or 5-ALA), hexyl levulinate (HAL) or methyl aminolaevulinate (MAL).

## Patentansprüche

1. Endoskop zur Untersuchung eines Körperhohlraums umfassend Gewebe und Körperfluide, wobei das Endoskop ein Beleuchtungssystem für endoskopische Anwendungen im Körperhohlraum umfasst, welches Beleuchtungssystem Folgendes umfasst:
- eine monochromatische Lichtquelle mit einer vorgegebenen zentralen Wellenlänge zwischen 500 und 550 nm,
- einen optischen Transmissionsweg, der dafür eingerichtet ist, von der Lichtquelle ausgehendes Licht zu einem endoskopischen Bereich der Untersuchung zu leiten, und
- mindestens eine optische Bandsperre,
wobei das Endoskop weiter Mittel zum Leiten von Licht von der monochromatischen Lichtquelle gegen mindestens einen Teil des Gewebes umfasst, welche Mittel den optischen Transmissionsweg umfassen, wobei das Endoskop dafür ausgeformt ist, mindestens einen Teil des bestrahlten Gewebes durch die Bandsperre auszugeben,
wobei die mindestens eine Bandsperre dafür eingerichtet ist, zumindest die Lichtquellenwellenlänge für einen Betrachter zu dämpfen, wobei die Bandsperre zum Dämpfen der Lichtquellenwellenlänge um mehr als 10 dB, vorzugsweise um mehr als 20 dB, vorzugsweise um mehr als 30 dB, vorzugsweise um mehr als 40 dB, vorzugsweise um mehr als 50 dB, am meisten bevorzugt um mehr als 60 dB, eingerichtet ist,
und
**dadurch gekennzeichnet, dass** die Lichtquelle die einzige Lichtquelle im Endoskop ist.

2. Endoskop nach Anspruch 1, wobei die Lichtquelle ein Laser ist, wie beispielsweise ein fasergekoppelter Laser, ein Faserlaser, ein Feststofflaser, ein diodengepumpter Feststofflaser, eine lichtemittierende Diode (LED) oder ein Halbleiterlaser, und die Lichtquelle, dafür eingerichtet ist, kontinuierliches Wellenlicht (CW-Licht) oder gepulstes Licht auszugeben.

3. Endoskop nach einem der vorgehenden Ansprüche, wobei der optische Transmissionsweg in einem Kabel oder einer Leitung mit einem Durchmesser von kleiner als 3 mm, bevorzugter kleiner als 2 mm, bevorzugter kleiner als 1,5 mm, bevorzugter kleiner als 1 mm, bevorzugter kleiner als 0,9 mm, bevorzugter kleiner als 0,8 mm, bevorzugter kleiner als 0,7 mm, bevorzugter kleiner als 0,6 mm, bevorzugter kleiner als 0,5 mm, bevorzugter kleiner als 0,4 mm, bevorzugter kleiner als 0,3 mm, bevorzugter kleiner als 0,25 mm, inkorporiert ist.

4. Endoskop nach einem der vorgehenden Ansprüche, wobei das Sperrband der Bandsperre die Lichtquellenwellenlänge umfasst, vorzugsweise ist das Sperrband der Bandsperre an der Lichtquellenwellenlänge zentriert.

5. Endoskop nach einem der vorgehenden Ansprüche, wobei das Sperrband der Bandsperre kleiner als 20 nm, bevorzugter kleiner als 15 nm, bevorzugter kleiner als 12 nm, bevorzugter kleiner als 10 nm, bevorzugter kleiner als 8 nm, bevorzugter kleiner als 6 nm, bevorzugter kleiner als 4 nm, bevorzugter kleiner als 2 nm, ist.

6. Endoskop nach einem der vorgehenden Ansprüche, wobei die Bandsperre ein Sperrfilter, vorzugsweise ein Raman-Sperrfilter, ist.

7. Endoskop nach einem der vorgehenden Ansprüche, wobei die monochromatische Lichtquelle am proximalen Ende des Endoskops oder äußerlich angeordnet ist.

8. Endoskop nach einem der vorgehenden Ansprüche, weiter umfassend mindestens eine Kamera und/oder umfassend ein Array von Sensorelementen.

9. Endoskop nach Anspruch 8, wobei die Kamera und/oder das Array von Sensorelementen entweder
- am distalen Ende des Endoskops oder
- am proximalen Ende des Endoskops angeordnet sind.

10. Endoskop nach einem der vorgehenden Ansprüche, wobei die Lichtquelle eine LED ist, und wobei das Endoskop weiter ein Bandpassfilter zur Einengung der spektralen Ausgabe der LED umfasst.

11. Endoskop nach einem der vorgehenden Ansprüche zur photodynamischen Diagnostizierung von Blasenkrebs und/oder Nierenbeckentumor oder -Krebs, wobei die Lichtquelle dafür eingerichtet ist, Fluoreszenz eines exogenen Photosensibilisators anzuregen, wobei der exogene Photosensibilisator in präkanzerösen, bösartigen und/oder schnellwachsenden Zellen in der Blase und/oder im Nierenbecken akkumuliert wird, und wobei der exogene Photosensibilisator auf Porphyrinen, wie Hämatoporphyrin oder Protoporphyrin, wie beispielsweise Protoporphyrin IX (PPIX) basiert, und wobei der exogene Photosensibilisator mittels einer photosensibilisierenden Farbe basierend auf Hexaminolevulinat, 5-Aminolevulinsäure (ALA oder 5-ALA), Hexyllevulinat (HAL) oder Methylaminolaevulinat (MAL) verabreicht wird.

## Revendications

1. Endoscope pour l'examen d'une cavité corporelle comprenant un tissu et des fluides corporels, l'endoscope comprenant un système d'éclairage pour des applications endoscopiques dans la cavité corporel, ledit système d'éclairage comprenant:
- une source de lumière monochromatique ayant une longueur d'onde centrale prédéfinie entre 500 et 550 nm,
- un trajet de transmission optique adapté pour guider la lumière provenant de la source de lumière à une région endoscopique d'examen, et
- au moins un filtre de réjection de bande optique,
l'endoscope comprenant en outre des moyens pour guider la lumière à partir de la source de lumière monochromatique vers au moins une partie du tissu, lesdits moyens comprenant le trajet de transmission optique, l'endoscope étant configuré de manière à afficher au moins une partie du tissu irradié à travers ledit filtre de réjection de bande,
ledit au moins un filtre de réjection de bande étant adapté de manière à atténuer au moins ladite longueur d'onde de la source de lumière pour un observateur, le filtre de réjection de bande étant adapté de manière à atténuer au moins ladite longueur d'onde de la source de lumière par plus de 10 dB, préférablement par plus de 20 dB, préférablement par plus de 30 dB, préférablement par plus de 40 dB, préférablement par plus de 50 dB, plus préférablement par plus de 60 dB,
et
**caractérisé en ce que** ladite source de lumière est la seule source de lumière dans l'endoscope.

2. Endoscope selon la revendication 1, dans lequel ladite source de lumière est un laser, tel qu'une fibre couplée au laser, un laser à fibre, un laser à l'état solide, un laser à l'état solide à pompage par diode, une diode à émission de lumière (DEL) ou un laser à semi-conducteur, et ladite source de lumière adaptée de manière à émettre une lumière à onde continue (CW, continuous wave) ou la lumière pulsée.

3. Endoscope selon l'une quelconque des revendications précédentes, dans lequel le trajet de transmission optique est incorporé dans un câble ou un fil ayant un diamètre inférieur à 3 mm, plus préférablement inférieur à 2 mm, plus préférablement inférieur à 1,5 mm, plus préférablement inférieur 1 mm, plus préférablement inférieur à 0,9 mm, plus préférablement inférieur à 0,8 mm, plus préférablement inférieur à 0,7 mm, plus préférablement inférieur à 0,6 mm, plus préférablement inférieur à 0,5 mm, plus préférablement inférieur à 0,4 mm, plus préférablement inférieur à 0,3 mm, plus préférablement inférieur à 0,25 mm.

4. Endoscope selon l'une quelconque des revendications précédentes, dans lequel la bande de réjection du filtre de réjection de bande comprend la longueur d'onde de la source lumineuse, de préférence la bande de réjection du filtre de réjection de bande étant centrée sur la longueur d'onde de la source lumineuse.

5. Endoscope selon l'une quelconque des revendications précédentes, dans lequel la bande de réjection du filtre est inférieure à 20 nm, plus préférablement inférieure à 15 nm, plus préférablement inférieure à 12 nm, plus préférablement inférieure à 10 nm, plus préférablement inférieure à 8 nm, plus préférablement inférieure à 6 nm, plus préférablement inférieure à 4 nm, plus préférablement inférieure à 2 nm.

6. Endoscope selon l'une quelconque des revendications précédentes, dans lequel le filtre de réjection de bande est un filtre coupe-bande, de préférence un filtre coupe-bande Raman.

7. Endoscope selon l'une quelconque des revendications précédentes, dans lequel la source de lumière monochromatique est située à l'extrémité proximale de l'endoscope ou à l'extérieur de celui-ci.

8. Endoscope selon l'une quelconque des revendications précédentes, comprenant en outre au moins une caméra et/ou comprenant un relais d'éléments de capteur.

9. Endoscope selon la revendication 8, dans lequel la caméra et/ou le relais d'éléments de capteur sont situés soit
- à l'extrémité distale de l'endoscope, soit
- à l'extrémité proximale de l'endoscope.

10. Endoscope selon l'une quelconque des revendications précédentes, dans lequel ladite source de lumière est une DEL, et dans lequel l'endoscope en outre comprend un filtre passe-bande pour rétrécir la sortie spectrale de la DEL.

11. Endoscope selon l'une quelconque des revendications précédentes pour le diagnostic photodynamique du cancer de la vessie et/ou d'un tumeur ou cancer de bassinet, dans lequel la source de lumière est adaptée de manière à exciter la fluorescence d'un agent photosensibilisant exogène, dans lequel l'agent photosensibilisant exogène est accumulée dans les cellules précancéreuses, malignes et/ou à croissance rapide dans la vessie et/ou du bassinet du rein, dans lequel l'agent photosensibilisant exogène est basée sur des porphyrines, tels que l'hématoporphyrine ou protoporphyrine, tels que la protoporphyrine IX (PpIX), et dans lequel le composé photosensible est délivré à la zone d'examen au moyen d'un colorant photosensibilisant à base de hexaminolévulinate, 5-aminolévulinique (ALA ou 5-ALA), lévulinate hexyle ou méthyle aminolaevulinate (MAL).
